# EUROPEAN PATENT APPLICATION

(11) **EP 1 829 881 A1**
(43) Date of publication of application: **05.09.2007**
(21) Application number: 07250698.3
(22) Date of filing: 20.02.2007
(51) Int. Cl.: C07D 498/16, C07D 498/22, A61K 31/436, A61L 31/16, A61P 1/02, A61P 1/04, A61P 1/12, A61P 1/16, A61P 3/10, A61P 7/02, A61P 7/06, A61P 9/10, A61P 9/14, A61P 11/06, A61P 17/06, A61P 17/10, A61P 17/14, A61P 17/18, A61P 19/10, A61P 21/04, A61P 27/02, A61P 31/00, A61P 35/00, A61P 37/00, A61P 39/06, A61P 43/00

(54) **Combination of rapamycin and its tetrazole isomers and epimers, methods of making and using the same**

(30) Priority: 28.02.2006 US 364447
(71) Applicant: Cordis Corporation, Miami Lakes, FL 33014 (US)
(72) Inventor: Zhao, Jonathon Z., Belle Mead, NJ 08502 (US)
(74) Representative: Belcher, Simon James

(57) **Abstract**

Epimers and isomers of tetrazole-containing rapamycin analogs are immunomodulatory agents and are useful in the treatment of restenosis and immune and autoimmune diseases. Also disclosed are cancer-, fungal growth-, restenosis-, post- transplant tissue rejection- and immune- and autoimmune disease- inhibiting compositions and a method of inhibiting cancer, fungal growth, restenosis, post-transplant tissue rejection, and immune and autoimmune disease in a mammal. It is preferred to use a combination of native rapamycin and its tetrazole containing isomers and epimers. One particular preferred application of such a combination of rapamycin and its tetrazole containing isomers and epimers is in medicated devices and local vascular delivery wherein the stability and lipid solubility and subsequently diffusion through tissue and cell membranes of the tetrazole isomers and epimers are essential to the success of the combined rapamycin formulation.

## Description

The present invention relates to novel isomers and epimers of tetrazole containing rapamycin and 42-Epi rapamycin analogs and synthetic methods for the preparation thereof. More particularly, the present invention relates to semisynthetic isomers and epimers of tetrazole containing rapamycin analogs and 42-Epi rapamycin analogs, means for their preparation, pharmaceutical compositions comprising such compounds, and methods of treatment employing the same.

The compound cyclosporine (cyclosporin A) has found wide use since its introduction in the fields of organ transplantation and immunomodulation, and has brought about a significant increase in the success rate for transplantation procedures. Recently, several classes of macrocyclic compounds having potent immunomodulatory activity have been discovered. Okuhara et al., in European Patent Application No. 184, 162, published Jun. 11, 1986, discloses a number of macrocyclic compounds isolated from the genus Streptomyces, including the immunosuppressant FK- 506, a 23-membered macrocyclic lactone, which was isolated from a strain of S. tsukubaensis.

Other related natural products, such as FR-900520 and FR-900523, which differ from FK-506 in their alkyl substituent at C-21, have been isolated from S. hygroscopicus yakushimnaensis. Another analog, FR- 900525, produced by S. tsukubaensis, differs from FK-506 in the replacement of a pipecolic acid moiety with a proline group. Unsatisfactory side-effects associated with cyclosporine and FK-506 such as nephrotoxicity, have led to a continued search for imnmunosuppressant compounds having improved efficacy and safety, including an immunosupressive agent which is effective topically, but ineffective systemically (US-5,457,111).

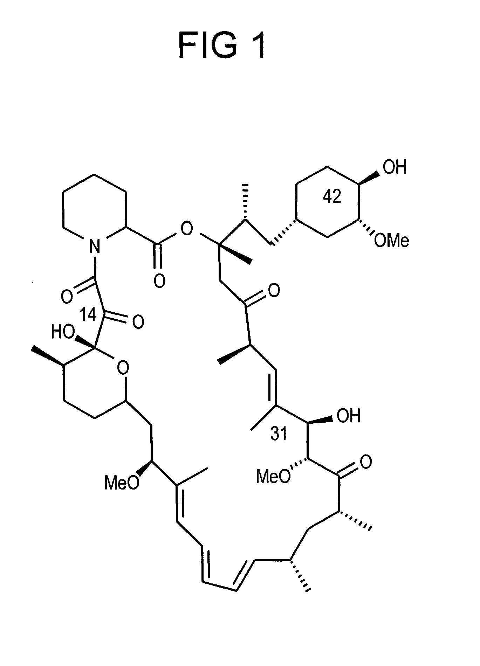

Rapamycin, illustrated in Figure 1, is a macrocyclic triene antibiotic produced by Streptomyces hygroscopicus, which was found to have antifungal activity, particularly against Candida albicans, both *in vitro* and *in vivo* (C. Vezina et al., J. Antibiot. 1975, 28, 721; S. N. Sehgal et al., J. Antibiot. 1975, 28, 727; H. A. Baker et al., J. Antibiot. 1978, 31, 539; U.S. Pat. No. 3,929,992; and U.S. Pat. No. 3,993,749).

Figure 1 illustrates a rapamycin structure as produced from a fermentation process.

Rapamycin alone (US-4,885,171) or in combination with picibanil (US-4,401,653) has been shown to have antitumor activity. In 1977, rapamycin was also shown to be effective as an immunosuppressant in the experimental allergic encephalomyelitis model, a model for multiple sclerosis; in the adjuvant arthritis model, a model for rheumatoid arthritis; and was shown to effectively inhibit the formation of IgE-like antibodies (R. Martel et al., Can. J. Physiol. Pharmacol., 1977, 55, 48).

The immunosuppressive effects of rapamycin have also been disclosed in FASEB, 1989, 3, 3411 as has its ability to prolong survival time of organ grafts in histoincompatible rodents (R. Morris, Med. Sci. Res., 1989, 17, 877). The ability of rapamycin to inhibit T-cell activation was disclosed by M. Strauch (FASEB, 1989, 3, 3411). These and other biological effects of rapamycin are reviewed in Transplantation Reviews, 1992, 6, 39-87.

Mono-ester and di-ester derivatives of rapamycin (esterification at positions 31 and 42) have been shown to be useful as antifungal agents (US-4,316,885) and as water soluble prodrugs of rapamycin (U.S. Pat. No. 4,650,803).

Fermentation and purification of rapamycin and 30-demethoxy rapamycin have been described in the literature (C. Vezina et al. J. Antibiot. (Tokyo), 1975, 28 (10), 721; S. N. Sehgal et al., J. Antibiot. (Tokyo), 1975, 28(10), 727; 1983, 36(4), 351; N. L. Pavia et al., J. Natural Products, 1991, 54(1), 167-177).

Numerous chemical modifications of rapamycin have been attempted. These include the preparation of mono- and di-ester derivatives of rapamycin (WO-A-92/05179), 27-oximes of rapamycin (EP-A-0467606); 42-oxo analog of rapamycin (US-5,023,262); bicyclic rapamycins (US-5,120,725); rapamycin dimers (US-5,120,727); silyl ethers of rapamycin (US-5,120,842); and arylsulfonates and sulfamates (US-5,177,203). Rapamycin was recently synthesized in its naturally occuring enantiomeric form (K. C. Nicolaou et al., J. Am. Chem. Soc., 1993, 115, 4419-4420; S. L. Schreiber, J. Am. Chem. Soc., 1993, 115, 7906-7907; S. J. Danishefsky, J. Am. Chem. Soc., 1993, 115, 9345-9346.

It has been known that rapamycin, like FK-506, binds to FKBP-12 (Siekierka, J. J.; Hung, S. H. Y.; Poe, M.; Lin, C. S.; Sigal, N. H. Nature, 1989,341, 755-757; Harding, M. W.; Galat, A.; Uehling, D. E.; Schreiber, S. L. Nature 1989, 341, 758-760; Dumont, F. J.; Melino, M. R.; Staruch, M. J.; Koprak, S. L.; Fischer, P. A.; Sigal, N. H. J. Immunol. 1990, 144, 1418-1424; Bierer, B. E.; Schreiber, S. L.; Burakoff, S. J. Eur. J. Immunol. 1991, 21, 439-445; Fretz, H.; Albers, M. W.; Galat, A.; Standaert, R. F.; Lane, W. S.; Burakoff, S. J.; Bierer, B. E.; Schreiber, S. L. J. Am. Chem. Soc. 1991, 113, 1409-1411). Recently it has been discovered that the rapamycin/FKBP-12 complex binds to yet another protein, which is distinct from calcineurin, the protein that the FK- 506/FKBP-12 complex inhibits (Brown, E. J.; Albers, M. W.; Shin, T. B.; Ichikawa, K.; Keith, C. T.; Lane, W. S.; Schreiber, S. L. Nature 1994, 369, 756-758; Sabatini, D. M.; Erdjument-Bromage, H.; Lui, M.; Tempest, P.; Snyder, S. H. Cell, 1994, 78, 35-43).

One recent example of a rapamycin analog is a tetrazole containing rapamycin analog (US-6,015,815, US-6,015,815, and US-6,329,386). In this rapamysscin analog, the tetrazole heterocyclic ring is used to replace the hydroxyl group to effect the analog.

Although some of these tetrazole containing rapamycin analogs exhibit immunosuppressive activity, anti-restenotic activities in suppressing the migration and growth of vascular smooth muscles, especially used in a stent coating, the need remains for more tetrazole containing rapamycin analogs and 42-Epi rapamycin analogs which possess a different polarity of the overall compound, which may subsequently exhibit different stability and solubility properties in a formulation. These structurally different isomers (region-isomer), and optical isomers and epimers are distinct chemical compounds different from the one described by US-6,015,815, US-6,329,386, and US-6,890,546. They also require additional fermentation or synthetic methods to prepare. Their unique polarity and optical properties may enable them to behave differently both *in vitro* and *in vivo*, and may translate into different metabolic rate and overall efficacy of a formulation, especially used in a local delivery or a drug device combination. These new isomers and epimers of tetrazole containing rapamycin analogs and 42-Epi rapamycin analogs may also possess potentially enhanced resistance to oxidative forces and better stability in a formulation through the tetrazole structure.

Accordingly, an object of the present invention is to provide novel semisynthetic isomers and epimers of tetrazole containing rapamycin analogs and 42-Epi rapamycin analogs. The starting materials may be prepared by either fermentation methods or synthetic methods.

In accordance with one aspect, the present invention is directed to the synthesis and use of these compounds with a rapamycin represented by the structural formulae illustrated in Figure 2 to Figure 7:
Figure 2 shows a 15-isomer of 42-tetrazole containing rapamycin analog (15 isomer of Zotarolimus).
Similarly, in accordance with another aspect, a compound of the present invention may contain different N substitution pattern(s) at 42- positions of a rapamycin, as illustrated in Figure 3.
Figure 3 shows a second 15-isomer of a 42 tetrazole containing rapamycin analog.

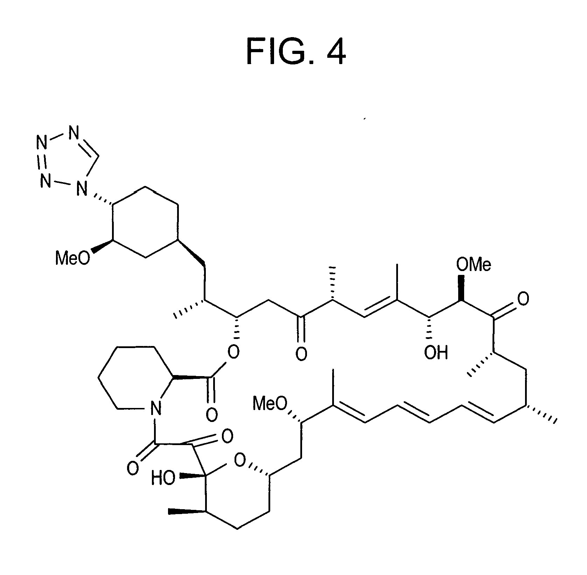

In accordance with another aspect, a compound of the present invention may be an epimer form of the compound illustrated in Figure 1 as shown in Figure 4.

Figure 4 shows another isomer of a 42 tetrazole containing Epi rapamycin analog (42 Epi- Zotarolimus).

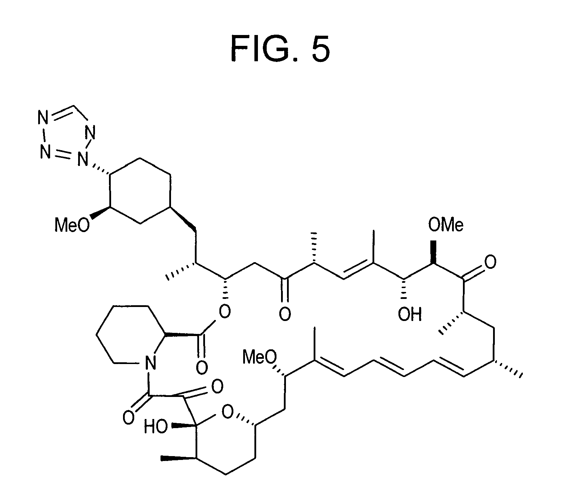

In accordance with another aspect, a compound of the present invention may be a tetrazole isomer form of the compound illustrated in Figure 1, as shown in Figure 5.

Figure 5 shows another isomer of a 42 tetrazole containing rapamycin analog.

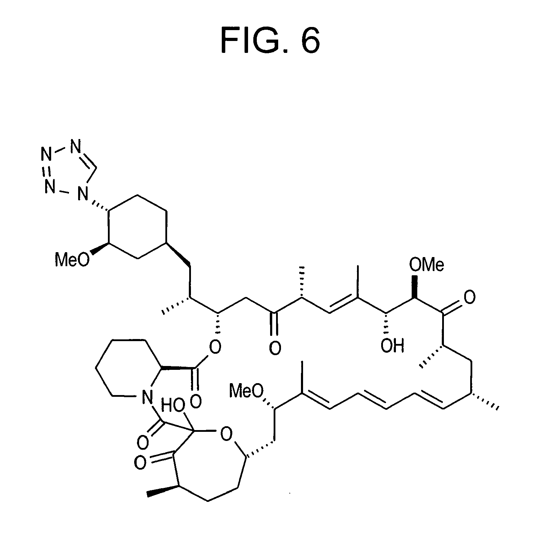

In accordance with another aspect, a compound of the present invention may be a tetrazole isomer form of the compound illustrated in Figure 1, as shown in Figure 6.

Figure 6 shows a 15 isomer of a tetrazole containing Epi rapamycin analog (15 isomer of Epi Zotarolimus).

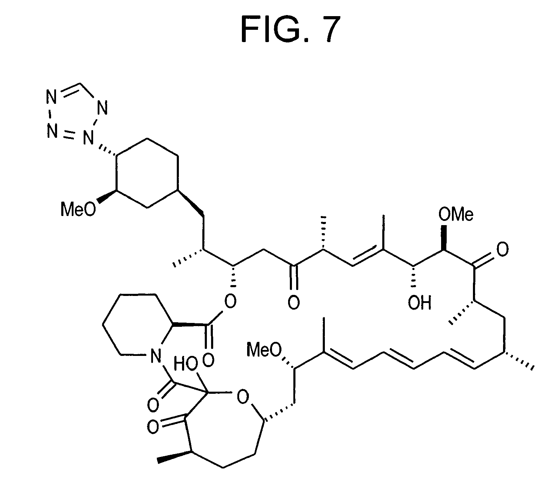

In accordance with another aspect, a compound of the present invention may be a tetrazole isomer form of the compound illustrated in Figure 1, as shown below in Figure 7.

Figure 7 shows a 15 isomer of a tetrazole containing rapamycin analog.

Another object of the present invention is to provide synthetic processes for the preparation of such compounds from starting materials obtained by fermentation, as well as chemical intermediates useful in such synthetic processes.

A further object of the present invention is to provide pharmaceutical compositions containing, as an active ingredient, at least one of the above compounds. The compounds disclosed in the present invention may be used in various pharmaceutical formulations such as oral liquids, oral suspension, intravenous injection, local intravascular injection, adventitial injection through a catheter, diffusion balloon catheter, a perivascular wrap device or any other suitable device.

Yet another object of the present invention is to provide a method of treating a variety of disease states, including restenosis, post- transplant tissue rejection, immune and autoimmune dysfunction, fungal growth, and cancer.

In addition, the compounds of the present invention may be employed as a solution, cream, or lotion by formulation with pharmaceutically acceptable vehicles containing 0.1- 5 percent and preferably 2 percent, of an active compound which may be administered to an area with fungal infection.

Embodiments of the invention will now be described by way of example with reference to the accompanying drawings, in which:
FIG.1 illustrates a rapamycin structure in accordance with the present invention.

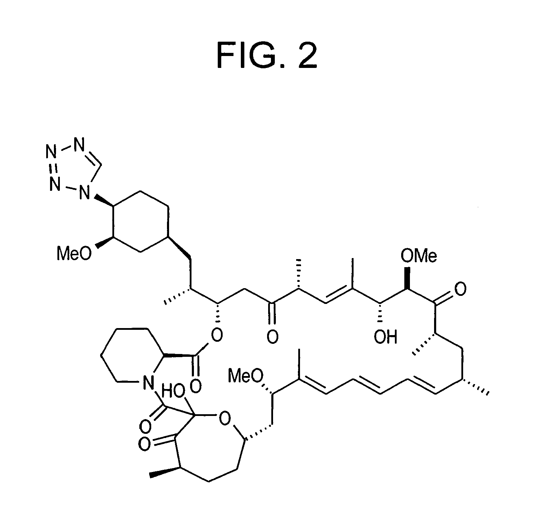

FIG.2 illustrates a 15-isomer of 42-tetrazole containing rapamycin analog (15 isomer of Zotarolimus).

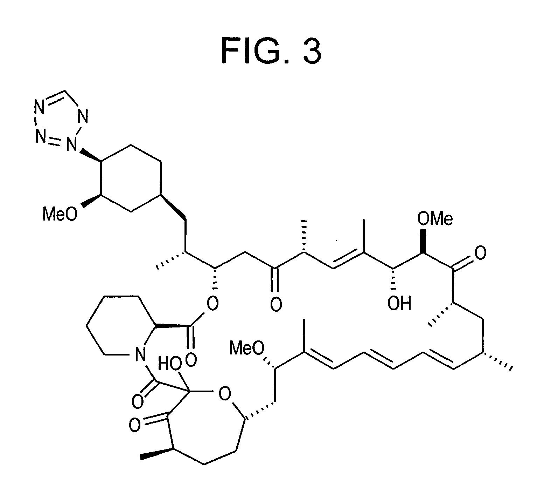

FIG.3 illustrates a second 15-isomer of a 42 tetrazole containing rapamycin analog.
FIG.4 illustrates another isomer of a 42 tetrazole containing Epi rapamycin analog (42 Epi- Zotarolimus).
FIG.5 illustrates another isomer of a 42 tetrazole containing rapamycin analog.
FIG.6 illustrates a 15 isomer of a tetrazole containing Epi rapamycin analog (15 isomer of Epi Zotarolimus).
FIG.7 illustrates a 15 isomer of a tetrazole containing rapamycin analog.

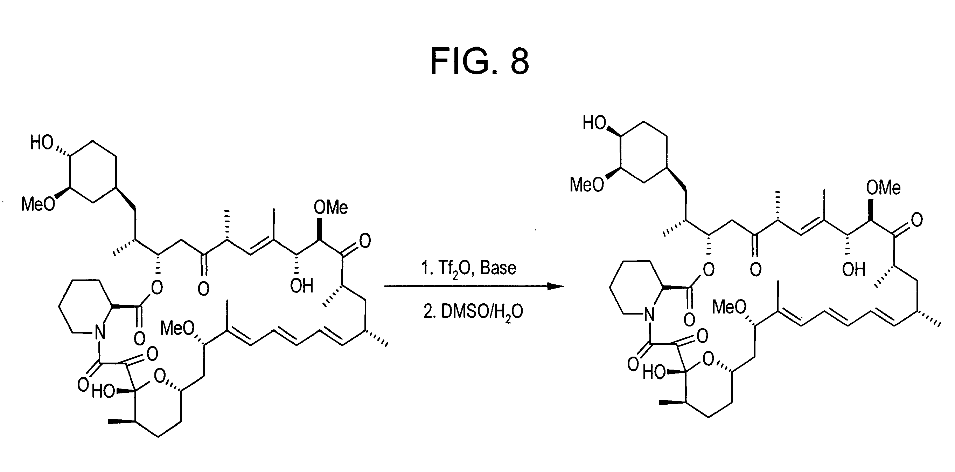

Figure 8 illustrates an exemplary reaction scheme to convert a rapamycin to its 42-Epi-rapamycin form in accordance with the present invention.

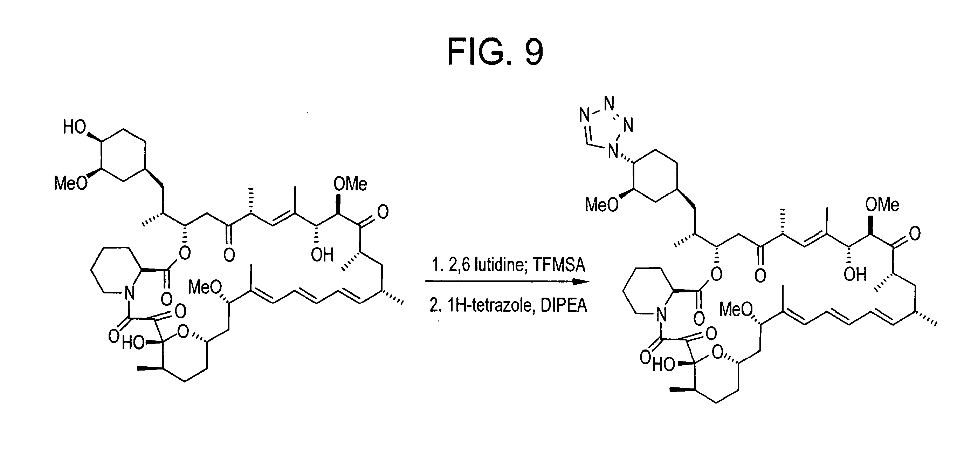

Figure 9 illustrates an exemplary reaction scheme to convert a 42-Epi-rapamycin to its tetrazole containing rapamycin analog in accordance with the present invention.

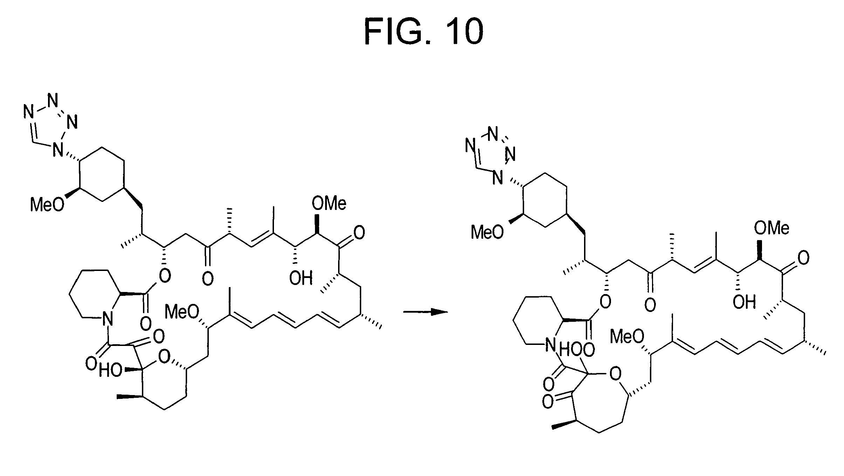

Figure 10 shows an exemplary reaction scheme to convert a tetrazole containing 42-Epi-rapamycin to its isomer in accordance with the present invention.

### Definition of Terms

The term "prodrug," as used herein, refers to compounds which are rapidly transformed *in vivo* to the parent compound of the above formula, for example, by hydrolysis in blood. A thorough discussion is provided in T. Higuchi and V. Stella, "Pro-drugs as Novel Delivery Systems," Vol. 14 of the A.C.S. Symposium Series, and in Edward B. Roche, ed., "Bioreversible Carriers in Drug Design," American Pharmaceutical Association and Pergamon Press, 1987, both of which are hereby incorporated by reference.

The term "pharmaceutically acceptable prodrugs," as used herein, refers to those prodrugs of the compounds of the present invention which are, within the scope of sound medical judgement, suitable for use in contact with the tissues of humans and lower mammals without undue toxicity, irritation, and allergic response, are commensurate with a reasonable benefit/risk ratio, and are effective for their intended use, as well as the zwitterionic forms, where possible, of the compounds of the invention. Particularly preferred pharmaceutically acceptable prodrugs of the present invention are prodrug esters of the C-31 hydroxyl group of compounds of the present invention.

The term "prodrug esters," as used herein, refers to any of several ester-forming groups that are hydrolyzed under physiological conditions. Examples of prodrug ester groups include acetyl, ethanoyl, pivaloyl, pivaloyloxymethyl, acetoxymethyl, phthalidyl, methoxymethyl, indanyl, and the like, as well as ester groups derived from the coupling of naturally or unnaturally-occuring amino acids to the C-31 hydroxyl group of compounds of the present invention.

The term "isomer" as used herein, refers to a compound having the identical chemical formula but different structural or optical configurations.

The term "epimer" as used herein, refers to a compound having the identical chemical formula but a different optical configuration at a particular position. In the case of a rapamycin, a 42-Epi rapamycin refers to the compound that has the opposite optical rotation compared to the rapamycin obtained by a fermentation process.

The term "I5-isomer" as used herein, refers to the analog of rapamycin that contains a 7-member ring at the 15-position as opposed to a regular rapamycin obtained from a fermentation process which contains a six-member ring. This kind of conversion is also called "tautomerization". The I5-isomer" as used herein, may also be referred to as a 15 tautomer of a rapamycin.

### Preparation of Compounds

The compounds and processes of the present invention will be better understood in connection with the following synthetic schemes which illustrate the methods by which the compounds of the invention may be prepared.

The compounds of the present invention may be prepared by a variety of synthetic routes.

As shown in Figure 8, rapamycin from the fermentation process may be converted to its 42-Epi rapamycin form. Other methods for the conversion reported in the literature, such as US-5,525,610 may be used to effect the conversion. Some intermediates, for example, 42-keto may be prepared from rapamycin and further converted to 42-Epi rapamycin as well, utilizing the method described in US-5,525,610.

Figure 8 illustrates an exemplary reaction scheme to convert a rapamycin to its 42-Epi-rapamycin form.

Once the 42- Epi rapamycin is obtained, it may be further converted to a tetrazole substituted rapamycin using a known method such as the one report in US-6,015,815. The process shown in Figure 9 may be achieved in two steps: first by triflating the hydroxyl group, followed by substitution with a 1 H-tetrazole. The process reverses the optical orientation of the substitute at the 42-position. The process also generates a mixture of two main substitutes (two region-isomers) such as shown in Figures 4 and 5. A regular preparatory LC column may be used to separate these two region-isomers since there exists a large difference in their polarity. The synthetics process may also give rise to a small amount of their respective optical isomers (epimers of the compounds shown in Figures 4 and 5). A optical chiral column may be needed to further separating the epimers of the compounds shown in Figures 4 an 5.

Figure 9 illustrates an exemplary reaction scheme to convert a 42-Epi-rapamycin to its tetrazole containing rapamycin analog (42 Epi Zotarolimus).

An exemplary process of converting a tetrazole containing rapamycin analog to its isomer (or tautmer) at the15 position is illustrated in Figure 10. The conversion may be achieved using the methods disclosed in US-A-2005/0014777. The conditions may be varied to increase the yield of isomer conversion.

Figure 10 illustrates an exemplary reaction scheme to convert a tetrazole containing 42-Epi-rapamycin (42 Epi Zotarolimus) to its 15 isomer.

### Methods of Treatment

The compounds of the present invention, including those specified in the examples, possess immunomodulatory activity in mammals (especially humans). As immunosuppressants, the compounds of the present invention are useful for the treatment and prevention of immune-mediated diseases such as the resistance by transplantation of organs or tissue such as heart, kidney, liver, medulla ossium, skin, cornea, lung, pancreas, intestinum tenue, limb, muscle, nerves, duodenum, small-bowel, pancreatic-islet-cell, and the like; graft-versus-host diseases brought about by medulla ossium transplantation; autoimmune diseases such as rheumatoid arthritis, systemic lupus erythematosus, Hashimoto's thyroiditis, multiple sclerosis, myasthenia gravis, type I diabetes, uveitis, allergic encephalomyelitis, glomerulonephritis, and the like. Further uses include the treatment and prophylaxis of inflammatory and hyperproliferative skin diseases and cutaneous manifestations of immunologically-mediated illnesses, such as psoriasis, atopic dermatitis, contact dermatitis and further eczematous dermatitises, seborrhoeis dermatitis, lichen planus, pemphigus, bulious pemphigoid, epidermolysis buliosa, urticaria, angioedemas, vasculitides, erythemas, cutaneous eosinophijias, lupus erythematosus, acne and alopecia areata; various eye diseases (autoimmune and otherwise) such as keratoconjunctivitis, vernal conjunctivitis, uveitis associated with Behcet's disease, keratitis, herpetic keratitis, conical cornea, dystrophia epithelialis corneae, corneal leukoma, and ocular pemphigus. In addition reversible obstructive airway disease, which includes conditions such as asthma (for example, bronchial asthma, allergic asthma, intrinsic asthma, extrinsic asthma and dust asthma), particularly chronic or inveterate asthma (for example, late asthma and airway hyper-responsiveness), bronchitis, allergic rhinitis, and the like are targeted by compounds of the present invention. Inflammation of mucosa and blood vessels such as gastric ulcers, vascular damage caused by ischemic diseases and thrombosis. Moreover, hyperproliferative vascular diseases such as intimal smooth muscle cell hyperplasia, restenosis and vascular occlusion, particularly following biologically- or mechanically-mediated vascular injury, could be treated or prevented by the compounds of the invention. Other treatable conditions include but are not limited to ischemic bowel diseases, inflammatory bowel diseases, necrotizing enterocolitis, intestinal inflammations/allergies such as Coeliac diseases, proctitis, eosinophilic gastroenteritis, mastocytosis, Crohn's disease and ulcerative colitis; nervous diseases such as multiple myositis, Guillain- Barre syndrome, Meniere's disease, polyneuritis, multiple neuritis, mononeuritis and radiculopathy; endocrine diseases such as hyperthyroidism and Basedow's disease; hematic diseases such as pure red cell aplasia, aplastic anemia, hypoplastic anemia, idiopathic thrombocytopenic purpura, autoimmune hemolytic anemia, agranulocytosis, pernicious anemia, megaloblastic anemia and anerythroplasia; bone diseases such as osteoporosis; respiratory diseases such as sarcoidosis, fibroid lung and idiopathic interstitial pneumonia; skin disease such as dermatomyositis, leukoderma vulgaris, ichthyosis vulgaris, photoallergic sensitivity and cutaneous T cell lymphoma; circulatory diseases such as arteriosclerosis, atherosclerosis, aortitis syndrome, polyarteritis nodosa and myocardosis; collagen diseases such as scleroderma, Wegener's granuloma and Sjogren's syndrome; adiposis; eosinophilic fasciitis; periodontal disease such as lesions of gingiva, periodontium, alveolar bone and substantia ossea dentis; nephrotic syndrome such as glomerulonephritis; male pattern aleopecia or alopecia senilis by preventing epilation or providing hair germination and/or promoting hair generation and hair growth; muscular dystrophy; Pyoderma and Sezary's syndrome; Addison's disease; active oxygen-mediated diseases, as for example organ injury such as ischemia-reperfusion injury of organs (such as heart, liver, kidney and digestive tract) which occurs upon preservation, transplantation or ischemic disease (for example, thrombosis and cardiac infarction); intestinal diseases such as endotoxin-shock, pseudomembranous colitis and colitis caused by drug or radiation; renal diseases such as ischemic acute renal insufficiency and chronic renal insufficiency; pulmonary diseases such as toxinosis caused by lung-oxygen or drug (for example, paracort and bleomycins), lung cancer and pulmonary emphysema; ocular diseases such as cataracta, siderosis, retinitis, pigmentosa, senile macular degeneration, vitreal scarring and corneal alkali burn; dermatitis such as erythema multiforme, linear IgA ballous dermatitis and cement dermatitis; and others such as gingivitis, periodontitis, sepsis, pancreatitis, diseases caused by environmental pollution (for example, air pollution), aging, carcinogenesis, metastasis of carcinoma and hypobaropathy; diseases caused by histamine or leukotriene-C.sub.4 release; Behcet's disease such as intestinal-, vasculo- or neuro-Behcet's disease, and also Behcet's which affects the oral cavity, skin, eye, vulva, articulation, epididymis, lung, kidney and so on. Furthermore, the compounds of the invention are useful for the treatment and prevention of hepatic disease such as immunogenic diseases (for example, chronic autoimmune liver diseases such as autoimmnune hepatitis, primary biliary cirrhosis and sclerosing cholangitis), partial liver resection, acute liver necrosis (e.g. necrosis caused by toxin, viral hepatitis, shock or anoxia), B-virus hepatitis, non-A/non-B hepatitis, cirrhosis (such as alcoholic cirrhosis) and hepatic failure such as fulminant hepatic failure, late- onset hepatic failure and "acute-on-chronic" liver failure (acute liver failure on chronic liver diseases), and moreover are useful for various diseases because of their useful activity such as augmention of chemotherapeutic effect, cytomegalovirus infection, particularly HCMV infection, anti-inflammatory activity, sclerosing and fibrotic diseases such as nephrosis, scleroderma, pulmonary fibrosis, arteriosclerosis, congestive heart failure, ventricular hypertrophy, post-surgical adhesions and scarring, stroke, myocardial infarction and injury associated with ischemia and reperfusion, and the like.

Additionally, compounds of the present invention possess FK-506 antagonistic properties. The compounds of the present invention may thus be used in the treatment of immunodepression or a disorder involving immunodepression. Examples of disorders involving immunodepression include AIDS, cancer, fungal infections, senile dementia, trauma (including wound healing, surgery and shock) chronic bacterial infection, and certain central nervous system disorders. The immunodepression to be treated may be caused by an overdose of an immunosuppressive macrocyclic compound, for example derivatives of 12-(2-cyclohexyl-1- methylvinyl)-13, 19,21,27-tetramethyl-11,28-dioxa4-azatricyclo[22.3.1.0. sup.4.9] octacos-18-ene such as FK-506 or rapamycin. The overdosing of such medicants by patients is quite common upon their realizing that they have forgotten to take their medication at the prescribed time and may lead to serious side effects.

The ability of the compounds of the present invention to treat proliferative diseases may be demonstrated according to the methods described in Bunchman E T and C A Brookshire, Transplantation Proceed. 23 967-968 (1991); Yamagishi, et al, Biochem. Biophys. Res. Comm. 191 840-846 (1993); and Shichiri, et al., J. Clin. Invest. 87 1867-1871 (1991). Proliferative diseases include smooth muscle proliferation, systemic sclerosis, cirrhosis of the liver, adult respiratory distress syndrome, idiopathic cardiomyopathy, lupus erythematosus, diabetic retinopathy or other retinopathies, psoriasis, scleroderma, prostatic hyperplasia, cardiac hyperplasia, restenosis following arterial injury or other pathologic stenosis of blood vessels. In addition, these compounds antagonize cellular responses to several growth factors, and therefore possess antiangiogenic properties, making them useful agents to control or reverse the growth of certain tumors, as well as fibrotic diseases of the lung, liver, and kidney.

When used to treat restenosis following a balloon angioplasty or stent placement, the compounds of the present invention, and the native rapamycin, are thought to exhibit their therapeutic functions through the inhibition of the mammalian target of rapamycin or mTOR. They may also bind to FKBP receptors.

Aqueous liquid compositions of the present invention are particularly useful for the treatment and prevention of various diseases of the eye such as autoimmune diseases including, for example, conical cornea, keratitis, dysophia epithelialis corneae, leukoma, Mooren's ulcer, sclevitis and Graves' ophthalmopathy, and rejection of corneal transplantation. These liquid formulations may also be administered through adventitial or perivascular routes to treat restenosis or vulnerable plaque.

When used in the above or other treatments, a therapeutically effective amount of one of the compounds of the present invention may be employed in pure form or, where such forms exist, in pharmaceutically acceptable salt, ester or prodrug form. Alternately, the compound may be administered as a pharmaceutical composition containing the compound of interest in combination with one or more pharmaceutically acceptable excipients. The phrase "therapeutically effective amount" of the compound of the present invention means a sufficient amount of the compound to treat disorders, at a reasonable benefit/risk ratio applicable to any medical treatment. It will be understood; however, that the total daily usage of the compounds and compositions of the present invention will be decided by the attending physician within the scope of sound medical judgement. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of the present invention administered to a human or lower mamal may range from about 0.01 to about 10 mg/kg/day. For purposes of oral administration, more preferable doses may be in the range from about 0.001 to about 3 mg/kg/day. If desired, the effective daily dose may be divided into multiple doses for purposes of administration; consequently, single dose compositions may contain such amounts or submultiples thereof to make up the daily dose. Topical adminstration may involve doses ranging from 0.001 to 3 percent mg/kg/day, depending on the site of application. When administered locally to treat restenosis and vulnerable plaque, the dose may range from about 1 microgram/mm stent length to about 100 microgram/mm stent length.

### Pharmaceutical Compositions

The pharmaceutical compositions of the present invention comprise a compound and a pharmaceutically acceptable carrier or excipient, which may be administered orally, rectally, parenterally, intracisternally, intravaginally, intraperitonealry, topically (as by powders, ointments, drops or transdermal patch), bucally, or as an oral or nasal spray. The phrase "pharmaceutically acceptable carrier" means a non-toxic solid, semi-solid or liquid filler, diluent, encapsulating material or formulation auxiliary of any type. The term "parenteral," as used herein, refers to modes of administration which include intravenous, intramuscular, intraperitoneal, intrasternal, subcutaneous and intraarticular injection and infusion.

Pharmaceutical compositions of the present invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

The compositions of the present invetnion may also contain adjuvants such as preservatives, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternately, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release may be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations may be sterilized, for example, by filtration through a bacterial-retaining filter, or by incorporating sterilizing agents in the form of sterile solid compositions which may be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fllers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft, semi-solid and hard-filled gelatin capsules or liquid- filled capsules using such excipients as lactose or milk sugar as well as high molecular weight polyethylene glycols and the like.

The solid dosage forms of tablets, dragees, capsules, pills, and granules may be prepared with coatings and shells such as enteric coatings and other coatings well known in the pharmaceutical formulating art. They may optionally contain opacifying agents and may also be of a composition that they release the active ingredient(s) only, or preferentially, in a certain part of the intestinal tract, optionally, in a delayed manner. Examples of embedding compositions which may be used include polymeric substances and waxes.

The active compounds may also be in micro-encapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar--agar, and tragacanth, and mixtures thereof.

Topical administration includes administration to the skin or mucosa, including surfaces of the lung and eye. Compositions for topical administration, including those for inhalation, may be prepared as a dry powder which may be pressurized or non-pressurized. In non-pressurized powder compositions, the active ingredient in finely divided form may be used in an admixture with a larger-sized pharmaceutically acceptable inert carrier comprising particles having a size, for example, of up to 100 micrometers in diameter. Suitable inert carriers include sugars such as lactose. Desirably, at least 95 percent by weight of the particles of the active ingredient have an effective particle size in the range of 0.01 to 10 micrometers. Compositions for topical use on the skin also include ointments, creams, lotions, and gels.

Alternately, the composition may be pressurized and contain a compressed gas, such as nitrogen or a liquified gas propellant. The liquified propellant medium and indeed the total composition is preferably such that the active ingredient does not dissolve therein to any substantial extent. The pressurized composition may also contain a surface active agent. The surface active agent may be a liquid or solid non-ionic surface active agent or may be a solid anionic surface active agent. It is preferred to use the solid anionic surface active agent in the form of a sodium salt.

A further form of topical administration is to the eye, as for the treatment of immune-mediated conditions of the eye such as automimmue diseases, allergic or inflammatory conditions, and corneal transplants. The compound of the present invention is delivered in a pharmaceutically acceptable ophthalmic vehicle, such that the compound is maintained in contact with the ocular surface for a sufficient time period to allow the compound to penetrate the corneal and internal regions of the eye, as for example the anterior chamber, posterior chamber, vitreous body, aqueous humor, vitreous humor, cornea, iris/cilary, lens, choroid/retina and sclera. The pharmaceutically acceptable ophthalmic vehicle may, for example, be an ointment, vegetable oil or an encapsulating material.

Compositions for rectal or vaginal administration are preferably suppositories or retention enemas which may be prepared by mixing the compounds of the present invention with suitable non-irritating excipients or carriers such as cocoa butter, polyethylene glycol or a suppository wax which are solid at room temperature but liquid at body temperature and therefore melt in the rectum or vaginal cavity and release the active compound.

Compounds of the present invention may also be administered in the form of liposomes. As is known in the art, liposomes are generally derived from phospholipids or other lipid substances. Liposomes are formed by mono- or multilamellar hydrated liquid crystals that are dispersed in an aqueous medium. Any non-toxic, physiologically acceptable and metabolizable lipid capable of forming liposomes may be used. The compositions in accordance with the present invention, in liposome form may contain, in addition to a compound of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 et seq.

In addition to these uses of the tetrazole derived isomers and epimers of rapamycin, they may be further combined with the native rapamycin to achieve a potentially better pharmacokinetic profiles and product stability. Rapamycin has been proven to be a extremely potent cytostatic inhibitor of smooth muscle cell migration and proliferation following an angioplasty procedure. A combined rapamycin and one of its tetrazole derived isomers and epimers may retain much of the proven efficacy and gain additional product stability and potentially superior efficacy, both systemic and locally.

Compounds of the present invention may also be co-administered with one or more immunosuppressant agents. The immunosuppressant agents within the scope of the presentinvention include, IMURAN.RTM. azathioprine sodium, brequinar sodium, SPANIDIN.RTM. gusperimus trihydrochloride (also known as deoxyspergualin), mizoribine (also known as bredinin), CELLCEPT.RTM. mycophenolate mofetil, NEORAL. RTM. Cylosporin A (also marketed as different formulation of Cyclosporin A under the trademark SANDIMMUNE.RTM.), PROGRAF.RTM. tacrolimus (also known as FK-506), sirolimus and RAPAMUNE.RTM., leflunomide (also known as HWA-486), glucocorticoids, such as prednisolone and its derivatives, antibody therapies such as orthoclone (OKT3) and Zenapax.RTM., and antithymyocyte globulins, such as thymoglobulins.

The local delivery of drug/drug combinations from a stent or other implantable device has the following advantages; namely, the prevention of vessel recoil and remodeling through the scaffolding action of the stent and the prevention of multiple components of neointimal hyperplasia or restenosis as well as a reduction in inflammation and thrombosis. This local administration of drugs, agents or compounds to stented coronary arteries may also have additional therapeutic benefit. For example, higher tissue concentrations of the drugs, agents or compounds may be achieved utilizing local delivery, rather than systemic administration. In addition, reduced systemic toxicity may be achieved utilizing local delivery rather than systemic administration while maintaining higher tissue concentrations. Also in utilizing local delivery from a stent rather than systemic administration, a single procedure may suffice with better patient compliance. An additional benefit of combination drug, agent, and/or compound therapy may be to reduce the dose of each of the therapeutic drugs, agents or compounds, thereby limiting their toxicity, while still achieving a reduction in restenosis, inflammation and thrombosis. Local stent-based therapy is therefore a means of improving the therapeutic ratio (efficacy/toxicity) of anti-restenosis, anti-inflammatory, antithrombotic drugs, agents or compounds.

It is understood that the foregoing detailed description and accompanying examples are merely illustrative and are not to be taken as limitations upon the scope of the invention, which is defined solely by the appended claims. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the scope thereof.

## Claims

1. A method of treating systemic and local diseases in a mammal, which comprises administering to the mammal a therapeutically effective amount of a combination of rapamycin and a derivative of the following structure:

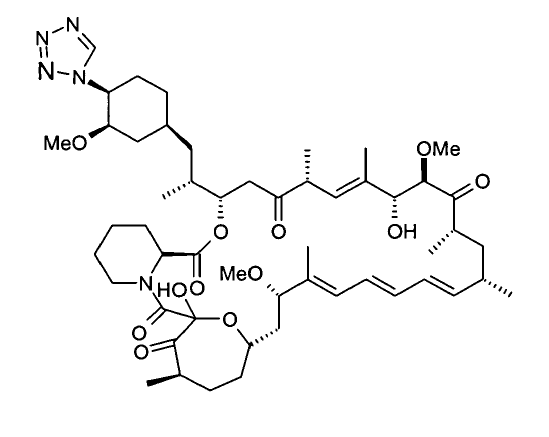

2. A method of treating systemic and local diseases in a mammal, which comprises administering to the mammal a therapeutically effective amount of a combination of rapamycin and a derivative of the following structure:

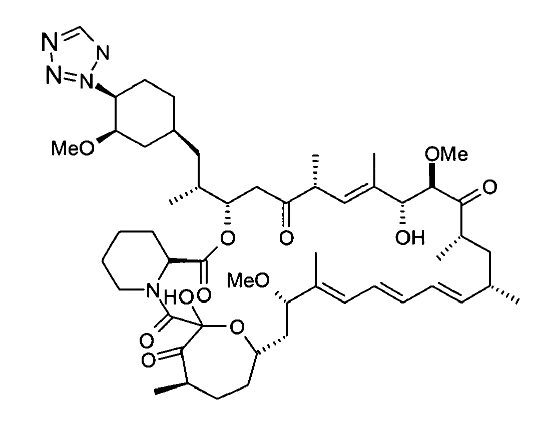

3. A method of treating systemic and local diseases in a mammal, which comprises administering to the mammal a therapeutically effective amount of a combination of rapamycin and a derivative of the following structure:

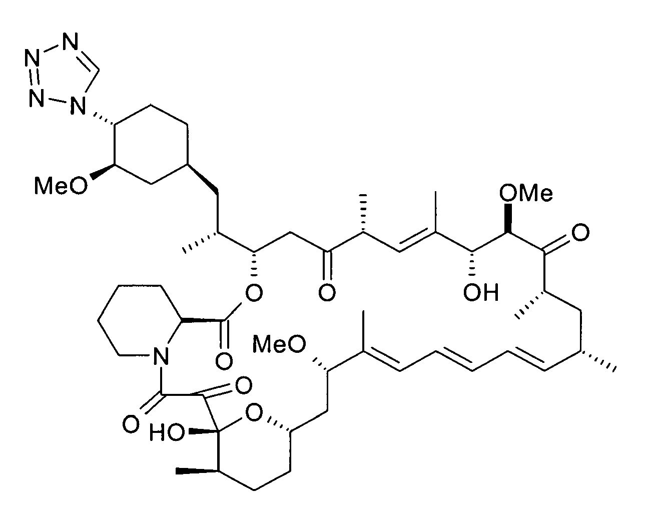

4. A method of treating systemic and local diseases in a mammal, which comprises administering to the mammal a therapeutically effective amount of a combination of rapamycin and a derivative of the following structure:

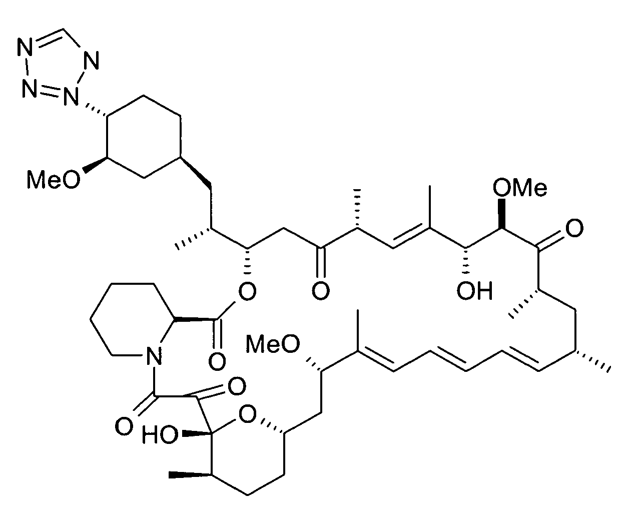

5. A method of treating systemic and local diseases in a mammal, which comprises administering to the mammal a therapeutically effective amount of a combination of rapamycin and a derivative of the following structure:

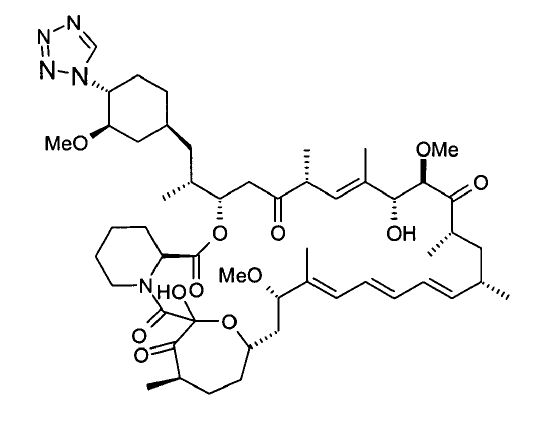

6. A method of treating systemic and local diseases in a mammal, which comprises administering to the mammal a therapeutically effective amount of a combination of rapamycin and a derivative of the following structure:

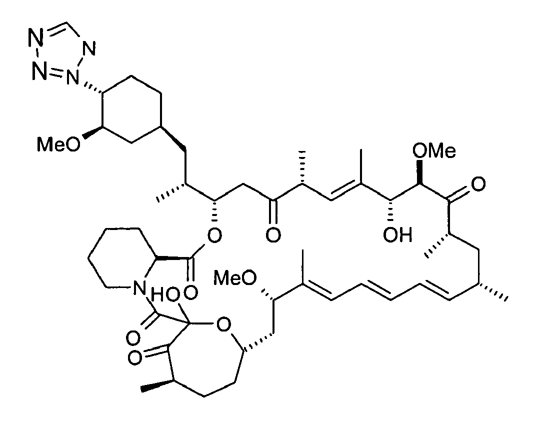

7. A method of treating neointimal growth associated with angioplasty balloon interventional procedures, which comprises administering to a mammal a therapeutically effective amount of a combination of rapamycin and a tetrazole derivative as claimed in any one of Claims 1-6.

8. A method of using of a combination of rapamycin and its tetrazole containing isomers and epimers as claimed in any one of Claims 1-6 in combination with an implantable device.

9. A method using rapamycin, one tetrazole containing isomer or epimer of rapamycin, as in any one of Claims 1-6, and a third therapeutic agent, systemically and locally to achieve therapeutic effects and enhanced stability.

10. A method using rapamycin, one tetrazole containing isomer or epimer of rapamycin, as in any one of Claims 1-6, and a third therapeutic agent, in an intravascular local injection to achieve therapeutic effects and enhanced stability.

11. A method using rapamycin, one tetrazole containing isomer or epimer of rapamycin, as in any one of Claims 1-6, and a third therapeutic agent, in an perivascular local injection to achieve therapeutic effects and enhanced stability.

12. A method using rapamycin, one tetrazole containing isomer or epimer of rapamycin, as in any one of claims 1-6, and a third therapeutic agent, in an adventitial local injection to achieve therapeutic effects and enhanced stability.

13. A method using rapamycin, one tetrazole containing isomer or epimer of rapamycin, as in any one of claims 1-6, and a third therapeutic agent, in coating a medical device.

14. A method using rapamycin, one tetrazole containing isomer or epimer of rapamycin, as in any one of claims 1-6, and a third therapeutic agent, in coating a stent.
